# EUROPÄISCHE PATENTSCHRIFT

(11) **EP 0 360 731 B1**
(45) Veröffentlichungstag und Bekanntmachung des Hinweises auf die Patenterteilung: **30.11.1994**
(21) Anmeldenummer: 89730200.6
(22) Anmeldetag: 21.09.1989
(51) Int. Cl.: A61F 13/08

(54) **Thrombose-Prophylaxe-Strumpf**
Stocking for thrombosis prevention
Bas pour prévenir la thrombose

(30) Priorität: 23.09.1988 DE 8812227 U
(43) Veröffentlichungstag der Anmeldung: 28.03.1990
(73) Patentinhaber: WEIHERMÜLLER & VOIGTMANN GMBH & CO KG, D-95449 Bayreuth (DE)
(72) Erfinder:
(74) Vertreter: Voigt, Günter, Dipl.-Ing.

(56) Entgegenhaltungen:
- DE-A- 2 458 251
- DE-U- 8 122 953
- FR-A- 2 213 762
- US-A- 3 546 900

## Beschreibung

Die Erfindung bezieht sich auf einen Thrombose-Prophylaxe-Strumpf gemäß Oberbegriff des Anspruchs 1.

Ein solcher Strumpf ist grundsätzlich bereits aus dem deutschen Gebrauchsmuster DE-U-8 122 953 bekannt.

Bei diesem vorbekannten Strumpf ist eine Fersenimitation an Stelle einer traditionell eingependelten Ferse vorgesehen. Die Fersenverstärkung dient dabei der Haltbarkeit. Auf diese Art und Weise soll die Scheuerfestigkeit der Ausbeulung erhöht werden.

Aus der US-A-3 546 900 ist ein rundgestrickter medizinischer Strumpf bekannt, bei dem sowohl elastische als auch unelastische Fäden verarbeitet werden. Von einem Plüsch- oder Flauschfaden, der ein Wundliegen verhindern soll, ist nicht die Rede.

Aus dem DE-U-1 929 623 ist bei einem rundgestrickten Kompressionsstrumpf mit Schußeinlage eine Ausbeulung vorgesehen, die - bedingt durch die Strickart - verstärkt wird. Die Ausbeulung wird in diesem Fall durch vergrößerte Maschen erreicht, die zur Erhöhung der Haltbarkeit wieder ausgefüllt werden.

Es sind auch bereits rotationssymmetrisch gestrickte Kompressionsstrümpfe mit im Ristbereich erweitertem Umfang bekannt, die wegen des unterbrechungslosen Strickvorganges preisgünstig sind. Nachteilig ist jedoch, daß sich die untere fußseitige Öffnung beim Tragen am Bein des Patienten oberhalb der Zehen befindet, so daß diese freiliegen, der Kompressionsstrumpf keine genügende Fixierung findet und nach oben wegrutschen kann (deutsches Gebrauchsmuster DE-U-7 415 893).

Aus der DE-A-2 458 251 und aus der US-A-3 603 116 sind medizinische Strümpfe bekannt, bei denen an Stelle einer traditionellen Pendelferse eine Fersenimitation vorgesehen ist. Hier werden Zusatzreihen für die Fersenausbeulung in einem Rundstrickstrumpf geschaffen, bei dem der zusätzliche Faden zur besseren Verriegelung des Fadenanfanges und des Fadenendes durch Maschenüberspringung verwendet wird. Diese Maschenüberspringung, auch "Flottliegen" genannt, ist in Figur 3 der DE-A-2 458 251 und in Figur 19 der US-A-3 603 116 dargestellt. Sie dient ausschließlich dem Problem der Verriegelung der zusätzlichen Reihe im Fersenbereich.

Aus dem deutschen Gebrauchsmuster DE-U-7 420 469 ist ein weiterer Kompressionsstrumpf bekannt, bei dem während der Herstellung auf der Rundstrickmaschine eine echte Ferse eingependelt wird, wodurch der Strumpf eine asymmetrische Form erhält und durch die bauchig ausgebildete Ferse eine Fixierung am Fuß des Patienten möglich ist. Da es jedoch fertigungstechnisch sehr schwierig ist, beim Einpendeln der Ferse auch einen Kompressions-Gummifaden in die Maschen einzulegen, ergibt sich beim Tragen des Strumpfes am Fuß des Patienten eine ungleichmäßige und medizinisch ungünstige Kompressionswirkung. Auch hier liegt die untere Öffnung des schlauchförmigen Gestrickes oberhalb der Zehen des Patienten.

Schließlich ist bei einem weiteren bekannten Thrombose-Prophylaxe-Strumpf die untere Öffnung im Zehenbereich als reduzierte Kontrollöffnung ausgebildet, so daß die Zehen des Patienten verdeckt sind und der Strumpf gleichzeitig fixiert ist. Im Ristbereich ist dieser Strumpf zunächst symmetrisch und mit vergrößerter Maschenweite gefertigt, wobei durch eine nachträgliche mechanisch-thermische Behandlung durch eine Dehnung der Maschen eine sogenannte falsche Ferse ausgearbeitet ist. Dadurch wird zwar im Ristbereich das Einpendeln einer echten Ferse erspart, jedoch erhöhen sich durch die nachträgliche Bearbeitung die Herstellungskosten (Versuche im Hause der Anmelderin).

Darüberhinaus sind zahlreiche weitere therapeutische Strümpfe bekannt, die elastomere Garne enthalten und einen Kompressionsdruck auf das Bein ausüben. Grundsätzlich soll der Druck, der von einem gut sitzenden Strumpf ausgeübt wird, vom Fußknöchel des Patienten bis zum Oberschenkel allmählich abnehmen.

Strümpfe dieser Art werden überwiegend im Krankenhausbereich bei liegenden Patienten vor, während oder nach Operationen verwendet. Dabei liegen die Patienten überwiegend auf dem Rücken, so daß der Fersenbereich als Auflage dient. Bei den bisher üblichen Strümpfen dieser Art ist es daher häufiger zu Rötungen und Druckstellen bis hin zu offenen Fersen bei den Patienten gekommen.

In Erkenntnis dieses Sachverhalts sind beim Gegenstand des deutschen Gebrauchsmusters DE-U-8 606 402 ein- oder beidseitig auf das Gestrick aufgesetzte und mit diesem durch eine Nähverbindung, eine Randverstrickung oder dergleichen verbundene Frotteegewebe vorgeschlagen worden, die für eine gute Feuchtigkeitsaufnahme durch das Material sorgen sollen und ein sogenanntes "Heißlaufen der Füße", Wundscheuern oder Wundlaufen vermeiden.

Solche auf das Grundgestrick aufgebrachte Frotteegewebe weisen jedoch den Nachteil auf, daß sie eine von dem Grundgestrick abweichende Elastizität besitzen und damit die Elastizität des Strumpfes in dem von ihnen belegten Bereich beeinflussen. Darüberhinaus kommt es wegen des unterschiedlichen Dehnungsverhaltens des Grundgestrickes einerseits und der aufgesetzten Frotteegewebe andererseits langfristig häufig zu Ablösungen des Frotteegewebes vom Grundgestrick.

Der Erfindung liegt die Aufgabe zugrunde, einen Thrombose-Prophylaxe-Strumpf zu schaffen, der zumindest im Fersenbereich auf der Innenseite eine dauerhafte weiche Auflage aufweist und gleichzeitig problemlos und günstig zu fertigen ist.

Die Lösung dieser Aufgabe erfolgt mit den Merkmalen des Anspruchs 1.

Vorteilhafte Weiterbildungen ergeben sich aus den Unteransprüchen.

Die Erfindung wird nachfolgend unter Bezugnahme auf die Zeichnungsfiguren beispielsweise erläutert. Es zeigen:
- Fig. 1: eine schematische Darstellung eines Thrombose-Prophylaxe-Strumpfes mit im Zehenbereich vorgesehener reduzierter Kontrollöffnung und im Fersen- und Ristbereich erweitertem Umfang sowie

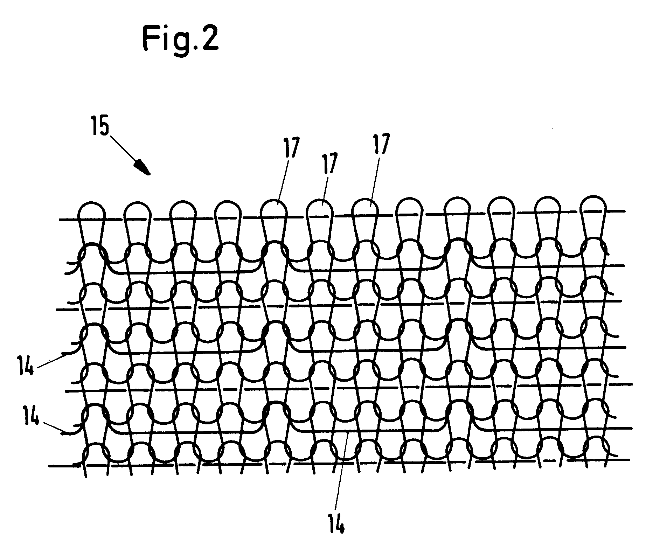

- Fig. 2: eine schematische Darstellung eines Gestrickes, bei dem der Plüsch- oder Flausch-Faden über mehrere Maschen des Gestrickes nicht abgebunden ist

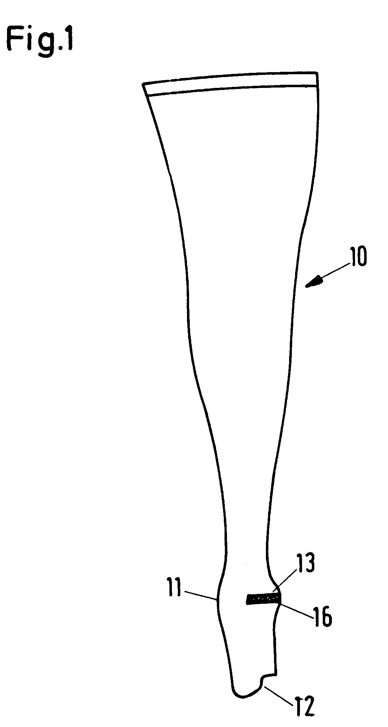

Der in Figur 1 schematisch dargestellte Strumpf 10 wird bei der Herstellung auf einer Rundstrickmaschine im Ristbereich 11 durch Vergrößerung der Maschenweite und durch angepaßte Verlängerung des in die Maschen eingelegten Kompressions-Schußfadens im Umfang vergrößert, wobei die übrigen Bereiche des Strumpfes 10 in bekannter Weise ausgeführt und mit Kompressions-Schußfäden versehen sind, die den medizinischen Anforderungen entsprechen. Unterhalb der Zehen ist der Kompressions-Schußfaden weggelassen und das üblicherweise schlauchförmige offene Ende durch eine Veränderung des Strickschemas als reduzierte Kontrollöffnung 12 ausgebildet, deren Rand noch mit einem Verstärkungsband eingefaßt sein kann. Beim Tragen des Strumpfes 10 sind die Zehen des Patienten oben verdeckt. Die Kontrollöffnung 12 liegt etwa an der Unterseite der Zehen.

Der mit erweitertem Umfang hergestellte Fersen- und Ristbereich 11 ist an dem der Ferse 13 zugeordneten Flächenabschnitt etwa zur Hälfte des Umfangs des Strumpfes zur Verstärkung mit einem zusätzlichen Faden gestrickt, der dem in den übrigen Bereichen des Strumpfes verwendeten Faden gleich oder ähnlich ist. Etwa in der Mitte des Flächenabschnitts der verstärkten Ferse 13 sind mit Hilfe eines optisch unterscheidbaren anderen Fadens etwa zwei bis 20 Maschenreihen eingependelt, die sich über etwa einen viertel bis über einen halben Umfang des Gestrickes erstrecken können. Im Vergleich zum Einpendeln der üblichen echten Ferse werden für das Einstricken des Streifens keine Pendelungen ausgeführt, so daß der übliche Rundstrickvorgang nicht unterbrochen wird. Wenn für die Herstellung des schmalen Streifens im verstärkten Flächenabschnitt der Ferse ein andersartiger, gegebenenfalls auch andersfarbiger Faden verwendet wird, ist der Fersenbereich des Strumpfes für das richtige Anlegen eindeutig festgelegt, während sich durch den eingearbeiteten schmalen Streifen im praktischen Gebrauch die Wirkung einer optisch ohne weiteres erkennbaren Quasi-Ferse ergibt.

Um eine weichere Auflage für die Ferse 13 und gegebenenfalls auch eine saugfähigere Auflage zu schaffen, wird im Bereich der Ferse 13 unter Verwendung eines zusätzlichen Stricksystems ein weicher und gegebenenfalls saugfähiger Plüsch- oder Flausch-Faden 14 in diesem Bereich des Gestrickes 15 angeordnet, der über mehrere Maschen "flott" liegend - das heißt nicht abgebunden - angeordnet ist. Dadurch ergeben sich im ungedehnten Zustand des Strumpfes 10 leichte Bögen oder Schlaufen des Plüsch-oder Flauschfadens 14 auf der Innenseite des Gestrickes 15. Dies ist erwünscht und wird erreicht durch eine spezielle Nadelauswahl während des Strickvorgangs. Der Plüsch- oder Flauschfaden 14 wird nicht wie das restliche Gestrick 15 von jeder Nadel erfaßt, sondern nur von jeder zweiten, dritten oder n-ten. Die Nadelauswahl erfolgt durch eine spezielle Mustereinrichtung an der Maschine. Dies kann über sogenannte Schnäbelstößer, Musterschieber und Zwischenschieber geschehen.

Der Plüsch- oder Flauschfaden 14 kann verschiedene Titer, d.h. Stärken, besitzen. Er sollte jedoch möglichst bauschig sein, um einen guten Abdeckeffekt und eine möglichst geringe Reibung zu gewährleisten. Außerdem muß der Plüsch- oder Flauschfaden 14 mit einem besonders dafür zugerichteten Fadenführer den Nadeln zugeführt werden, wobei Höhe und Position und damit der Einlaufwinkel des Fadens zur Nadelauswahl entscheidend sind. Auch die Bremsung und damit die Spannung des Fadens hat einen wesentlichen Einfluß auf die Schlingenbildung und wird über eine besonders konstruierte Fadenbremseinrichtung erzeugt.

Das Grundgestrick kann aus einem oder mehreren elastomeren Fäden oder aus Fäden mit elastomeren Bestandteilen bestehen. Auch der Plüsch- oder Flauschfaden 14 kann möglicherweise elastomere Bestandteile aufweisen. Es muß jedoch gewährleistet sein, daß die gewünschte Flauschigkeit des Plüsch- oder Flauschfadens dadurch nicht wesentlich beeinträchtigt wird.

Die weiter oben erwähnten Maßnahmen können auch bei sogenannten Stützstrümpfen vorgesehen werden.

Zusätzlich zu dem als Polsterung wirkenden Flausch erhält der Strumpf im Fersen- und Fuß-Rist-Bereich eine den Druck reduzierende blasenförmige Form. Dabei wird die in der Regel als Ferse 13 dienende Auswölbung des Strumpfes 10 an der Strumpfunterseite auch nach oben hin (Ristbereich) zu einer Blasenform ausgewölbt, so daß der Druck des Strumpfes in diesem Bereich (Ristbereich) verringert wird. Die von den Patienten als unangenehm empfundenen Druckfalten werden so weitgehend vermieden.

Da ein solcher Strumpf 10 sowohl an der Unterseite, d.h. an der Ferse 13, als auch an der Oberseite (Ristbereich 11) eine blasenförmige Ausbauchung aufweist, besteht die Gefahr, daß er nicht in der rechten Weise - sondern möglicherweise verdreht - angelegt wird. Hier dient ein farbiger Kennstreifen 16 als Orientierung. Die durch den farbigen Kennstreifen 16 gegebene Markierung verläuft von der Innenseite des Fußes über die Ferse zur Außenseite des Fußes und reicht so vorzugsweise über die Hälfte des Umfangs des Strumpfes in diesem Bereich.

## Patentansprüche

1. Thrombose-Prophylaxe-Strumpf mit abnehmender Kompression von distal nach proximal, wobei der Bereich der Ferse (13) und des Fußrists (11) jeweils bogenförmig ausgeformt sind und im Bereich der Ferse (13) ein zusätzlicher Faden weitgehend gleicher Elastizität wie der Faden des Grund-Gestricks (15) "flottliegend", d.h. nicht abgebunden, vorhanden ist, dadurch gekennzeichnet, daß der zusätzliche Faden in horizontaler Richtung ein Plüsch- oder Flausch-Faden (14) ist, der über ein, zwei oder mehr Maschen (17) des Gestricks (15) auf dessen Innenseite angeordnet ist.

2. Strumpf nach Anspruch 1, dadurch gekennzeichnet, daß der Plüsch- oder Flausch-Faden (14) ein gewisses Übermaß aufweist und demzufolge im ungedehnten Zustand des Strumpfes (10) auf dessen innenseitiger Oberfläche Bögen oder Schlaufen bildet.

3. Strumpf nach einem der Ansprüche 1 oder 2, dadurch gekennzeichnet, daß das Grund-Gestrick (15) aus einem oder mehreren elastomeren Fäden bzw. aus Fäden mit elastomeren Bestandteilen besteht.

4. Strumpf nach einem der Ansprüche 1 bis 3, dadurch gekennzeichnet, daß der Plüsch- oder Flausch-Faden (14) ebenfalls elastomere Bestandteile aufweist.

5. Strumpf nach einem der Ansprüche 1 bis 4, dadurch gekennzeichnet, daß der im Umfang vergrößerte Fersen- und Ristbereich (11) in dem der Ferse (13) zugeordneten Flächenabschnitt mit einem zusätzlichen, dem übrigen Gestrickfaden ähnlichen Faden verstärkt ist und - vorzugsweise in der Mitte des verstärkten Flächenabschnitts der Ferse - mit Hilfe eines optisch unterscheidbaren anderen Fadens zur Fixierung der Ferse in Richtung des Strumpfumfangs zusätzlich ein schmaler Streifen (16) in Höhe von mehreren Maschenreihen und in der Breite von einem Viertel bis zu einer Hälfte des Umfangs im Fersen- und Ristbereich (11) zur Kennzeichnung der Ferse (13) eingestrickt ist.

## Claims

1. Thrombosis prophylactic stocking with reducing compression from distal to proximal, wherein the region of the heel (13) and of the foot instep (11) are each formed arcuately and an additional thread of largely the same elasticity as the thread of the basic knitted structure (15) is "floatingly", i.e. not bound in, present in the region of the heel (13), characterised thereby that the additional thread in horizontal direction is a plush or fleecy thread (14) which is arranged over one, two or more stitches (17) of the knitted structure (15) on the inner side thereof.

2. Stocking according to claim 1, characterised thereby that the plush or fleecy thread (14) has a certain oversizing and consequently in the unextended state of the stocking (10) forms curves or loops on the inward surface thereof.

3. Stocking according to one of claims 1 or 2, characterised thereby that the basic knitted structure (15) consists of one or more elastomer threads or of threads with elastomer components.

4. Stocking according to one of claims 1 to 3, characterised thereby that the plush or fleecy thread (14) similarly has elastomer components.

5. Stocking according to one of claims 1 to 4, characterised thereby that the heel and instep region (11) enlarged in circumference is reinforced in the surface portion associated with the heel (13) by an additional thread similar to the remaining knitted structure thread and in addition a narrow strip (16) for marking of the heel is knitted in - preferably in the middle of the reinforced surface portion of the heel - with a height of several stitch rows and a width of one quarter to one half of the circumference in the heel and instep region (11) with the aid of a visually distinguishable other thread for the fixing of the heel in direction of the stocking circumference.

## Revendications

1. Bas pour la prophylaxie des thromboses, dans lequel la compression décroît de l'extrémité distale à l'extrémité proximale, cependant que les régions du talon (13) et du coude-pied (11) sont chacune réalisées en forme d'arc, et qu'il existe dans la région du talon (13) un fil supplémentaire qui est "lâche", c'est-à-dire non lié, et dont l'élasticité est dans une large mesure la même que celle du fil du tricot de base (15), caractérisé par le fait que le fil supplémentaire est un fil pelucheux ou à longs poils (14) qui est disposé dans la direction horizontale en passant sur une ou deux mailles (17) du tricot (15), ou plus, sur le côté intérieur de celui-ci.

2. Bas selon la revendication 1, caractérisé par le fait que le fil pelucheux ou à longs poils (14) présente un certain surdimensionnement qui forme en conséquence des arcs ou des boucles sur la surface du côté intérieur du bas (10) dans l'état où celui-ci n'est pas étiré.

3. Bas selon l'une des revendications 1 et 2, caractérisé par le fait que le tricot de base (15) est constitué par un ou plusieurs fils en élastomère ou, respectivement, par des fils qui comprennent des constituants en élastomère.

4. Bas selon l'une des revendications 1 à 3, caractérisé par le fait que le fil pelucheux ou à longs poils (14) comprend également des constituants en élastomère.

5. Bas selon l'une des revendications 1 à 4, caractérisé par le fait que la région du talon et du cou-de-pied (11) dont le pourtour est élargi est renforcée dans la portion de la surface associée au talon (13) par un fil supplémentaire qui est analogue au fil du reste du tricot, et que, pour marquer le talon (13), une bande étroite (16) est tricotée sur la hauteur de plusieurs rangées de mailles et sur une largeur allant du quart à la moitié du pourtour dans la région du talon et du cou-de-pied (11) - de préférence au milieu de la portion renforcée de la surface du talon - à l'aide d'un autre fil qui peut être distingué visuellement pour indiquer la position du talon dans la direction du pourtour du bas.
